(19) Europäisches Patentamt
European Patent Office
Office européen des brevets

(11) Veröffentlichungsnummer: **0 257 521 A2**

(12) # EUROPÄISCHE PATENTANMELDUNG

(21) Anmeldenummer: **87111937.6**

(51) Int. Cl.4: **C07D 309/28**, C07D 309/32

(22) Anmeldetag: **18.08.87**

(30) Priorität: **22.08.86 DE 3628576**

(43) Veröffentlichungstag der Anmeldung: **02.03.88 Patentblatt 88/09**

(84) Benannte Vertragsstaaten: **AT BE CH DE FR GB LI**

(71) Anmelder: **BASF Aktiengesellschaft**
**Carl-Bosch-Strasse 38**
**D-6700 Ludwigshafen(DE)**

(72) Erfinder: **Spiegler, Wolfgang, Dr.**
**Westpreussenstrasse 5**
**D-6520 Worms 27(DE)**
Erfinder: **Goetz, Norbert, Dr.**
**Schoefferstrasse 25**
**D-6520 Worms 1(DE)**
Erfinder: **Sauerwald, Manfred, Dr.**
**Gebhardtstrasse 16**
**D-6701 Roedersheim-Gronau(DE)**
Erfinder: **Dockner, Toni, Dr.**
**Grossgasse 6**
**D-6701 Meckenheim(DE)**
Erfinder: **Fischer, Rolf, Dr.**
**Bergstrasse 98**
**D-6900 Heidelberg(DE)**

(54) **3,4-Dihydro-2H-pyrane und ein Verfahren zu ihrer Herstellung.**

(57) 3,4-Dihydro-2H-pyrane I

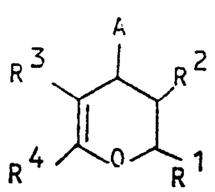
I

mit $R^1$, $R^2$, $R^3$, $R^4$ = H, $C_1$-bis $C_6$-Alkyl, wobei $R^3 \neq$ $C_2$-oder $C_4$-Alkyl, wenn A = $COOR^5$,
A = $COOR^5$, = $CHOR^5$ mit $R^5$ = $C_1$-bis $C_4$-Alkyl; $CH_2OR^6$ mit $R^6$ = $C_1$-bis $C_4$-Alkyl, Benzyl, ggf. substituiert durch $C_1$-bis $C_4$-Alkyl, Alkoxy oder Halogen, Formyl, $C_2$-bis $C_4$-Alkylcarbonyl oder Benzoyl, ggf. substituiert durch $C_1$-bis $C_4$-Alkyl oder Alkoxy oder Halogen
und ihre Herstellung durch Abspaltung von Alkoholen aus 2-Alkoxy-tetrahydropyranen II

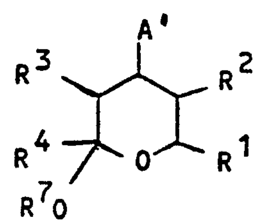
II

mit $R^7$ = $C_1$-bis $C_{18}$-Alkyl
A' = $COOR^5$, $CH(OR^5)_2$ oder $CH_2OR^6$, wobei $R^5$ und $R^6$ die in I genannte Bedeutung haben,
wobei man die Abspaltung durch Einleiten von II in flüssigem oder gasförmigem Zustand in ein hochsiedendes Mineralöl bei Temperaturen oberhalb des Siedepunkts des sich bildenden Dihydropyrans I durchführt, I gasförmig abzieht, das hochsiedende Mineralöl bei Anreicherung mit Nebenprodukten erneuert und das mit Nebenprodukten angereicherte hochsiedende Mineralöl abzieht.

EP 0 257 521 A2

# 3,4-Dihydro-2H-pyrane und ein Verfahren zu ihrer Herstellung

Die vorliegende Erfindung betrifft neue 3,4-Dihydro-2H-pyrane sowie ein Verfahren zu ihrer Herstellung durch Abspaltung von Alkoholen aus 2-Alkoxytetrahydropyranen.

Gemäß dem Stand der Technik, z.B. Houben-Weyl, Methoden der organischen Chemie, Bd. 6/4, 1966, Seite 85, werden 3,4-Dihydropyrane durch Erhitzen von 2-Alkoxy-tetrahydropyranen in Gegenwart starker Säuren oder Phosphorpentoxid in flüssiger Phase unter Abspaltung von Alkoholen erhalten. In der Regel liegen die Temperaturen bei 130 bis 200°C. Infolge dieser drastischen Bedingungen entstehen erhebliche Mengen an Nebenprodukten, z.B. durch Isomerisierung und Polymerisation des gebildeten Dihydropyrans und damit erhebliche Ausbeuteverluste.

In Journal of Organic Chemistry <u>44</u>, 366 (1979) wird ein besonders schonendes Verfahren zur Herstellung von 3,4-Dihydropyranen beschrieben. Danach wird das 2-Alkoxy-tetrahydropyran in Gegenwart katalytischer Mengen p-Toluolsulfonsäure auf ca. 160°C erwärmt und die Reaktionsprodukte Alkohol und Dihydropyran destillativ abgetrennt. Bei der Umsetzung von Ausgangsstoffen mit säureempfindlichen Resten verläuft jedoch selbst dieses Verfahren umbefriedigend, so erhält man z.B. ausgehend von 2-Methoxy-4-dimethoxymethyltetrahydropyran bereits bei unvollständigem Umsatz ein komplexes Reaktionsgemisch, in dem eine Vielzahl von Nebenprodukten (siehe Vergleichsbeispiel 4) auftreten.

Der Erfindung lag nun die Aufgabe zugrunde, 3,4-Dihydro-2H-pyrane mit bislang unbekanntem Substitutionsmuster, insbesondere mit säureempfindlichen Resten, zur Verfügung zu stellen und dabei einen vorteilhaften Syntheseweg zu beschreiten.

Demgemäß wurden 3,4-Dihydro-2H-pyrane der allgemeinen Formel I

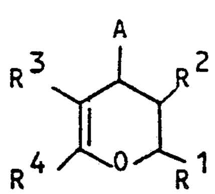 I

gefunden, in der die Reste $R^1$ bis $R^4$ gleich oder verschieden sind und für Wasserstoff oder einen $C_1$ bis $C_6$-Alkylrest stehen und A die Reste $COOR^5$ oder $=CH-OR^5$, in denen $R^5$ einen $C_1$-bis $C_4$-Alkylrest darstellt oder A den Rest $CH_2-OR^6$ bedeutet, in dem $R^6$ einen $C_1$-bis $C_4$-Alkylrest, einen Arylrest, der gegebenenfalls durch einen $C_1$-bis $C_4$-Alkyl-oder Alkoxyrest oder durch Halogen im Phenylring substituiert ist, den Formylreste, einen $C_2$-bis $C_4$-Alkylcarbonylrest oder einen Benzoylrest, der gegebenenfalls durch $C_1$-bis $C_4$-Alkyl-oder Alkoxyrest oder Halogen substituiert ist, darstellt, mit der Maßgabe, daß $R^3$ nicht für einen $C_2$-oder $C_4$-Alkylrest steht, wenn A den Rest $COOR^5$ bedeutet.

Weiterhin wurde ein Verfahren zur Herstellung von 3,4-Dihydro-2H-pyranen der Formel durch Abspaltung von Alkoholen $R^7OH$ aus 2-Alkoxy-tetrahydropyranen der Formel II

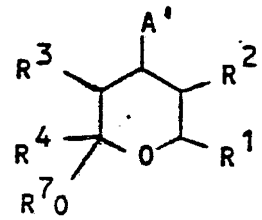 II,

in der die Reste $R^1$ bis $R^4$ gleich oder verschieden sind und für Wasserstoff oder einen $C_1$-bis $C_6$-Alkylrest stehen, A die für Verbindung I genannte Bedeutung hat, A' den Rest $COOR^5$, $CH(OR^5)_2$ oder $CH_2OR^6$ darstellt, wobei die Reste $R^5$ und $R^6$ die für Verbindung I genannte Bedeutung haben und der Rest $R^7$ für einen $C_1$-bis $C_{18}$-Alkylrest steht, gefunden, welches dadurch gekennzeichnet ist, daß man die Abspaltungsreaktion durch Einleiten des 2-Alkoxy-tetrahydropyrans II in flüssigem oder gasförmigem Zustand in ein hochsiedendes Mineralöl bei Temperaturen oberhalb des Siedepunkts des sich bildenden 3,4-Dihydropyrans I durchführt, das Dihydropyran I gasförmig abzieht, das hochsiedende Mineralöl bei Anreicherung mit Nebenprodukten erneuert und das mit Nebenprodukten angereicherte hochsiedende Mineralöl abzieht.

Die in den Verbindungen I und dementsprechend in den Ausgangsstoffen II angegebenen Reste $R^1$, $R^2$, $R^3$ und $R^4$ bedeuten unabhängig voneinander Wasserstoff oder verzweigte oder unverzweigte $C_1$-bis $C_6$-Alkylreste, z.B. Methyl-, Ethyl-, Propyl-, Isopropyl-, Butyl-, Isobutyl-, tert.-Butyl-, Pentyl-, 3-Methylbutyl-oder Hexylreste.

Der Rest A in der Formel I steht für eine funktionelle Gruppe $COOR^5$ $=CH-OR^5$ oder $CH_2-OR^6$, wobei $R^5$ einen $C_1$-bis $C_6$-Alkylrest darstellt und $R^6$ die Bedeutung von $R^5$ hat von darüber hinaus einen Benzylrest, der im Phenylring gegebenenfalls durch einen $C_1$-bis $C_4$-Alkyl-oder Alkoxyrest oder durch Halogen, z.B. Fluor, Chlor oder Brom substituiert ist, darstellt. Beispielsweise seien

der o-Methyl-, der p-Methoxy-, p-Butoxy-, der ortho-oder para-Chlor-oder Brombenzylrest genannt. Weiterhin steht $R^6$ für den Formylrest, einen $C_2$-bis $C_4$-Alkylcarbonylrest, z.B. einen Acetyl-oder Propionylrest sowie einen Benzoylrest, der gegebenenfalls durch $C_1$-bis $C_4$-Alkyl-oder Alkoxyrest oder durch Halogen, z.B. Chlor oder Brom, substituiert ist.

Beispielsweise seien folgende Reste A aufgeführt: Methoxycarbonyl, Ethoxycarbonyl, Propoxycarbonyl-, Isopropoxycarbonyl-, Butoxycarbonyl, Methoxy-, Ethoxy-, Propoxy-, Butoxy-und tert.-Butoxymethyliden, Methoxymethyl, Ethoxymethyl, Propoxymethyl, Isopropoxymethyl, Butoxymethyl, Isobutoxymethyl, tert.-Butoxymethyl, Benzyloxymethyl, Formyloxymethyl, Acetoxymethyl, Propionyloxymethyl, Benzoyloxymethyl, 4-Methylbenzoyloxymethyl, 4-Methoxybenzoyloxymethyl oder 4-Chlorbenzoyl-oxymethyl.

Im Ausgangsstoff II steht A' für den Rest $COOR^5$, $CH_2OR^6$, wobei die Reste $R^5$ und $R^6$ die in I genannte Bedeutung haben sowie anstelle des Restes $=CH\text{-}OR^5$ in I für den Rest $CH(OR^5)_2$, z.B. für Dimethoxy-, Diethoxy-, Dipropoxy-, Dibutoxymethyl.

Als Rest $R^7$ in Formel II kommen $C_1$-bis $C_{18}$-Alkylreste, insbesondere $C_1$-bis $C_8$-, vorzugsweise $C_1$-bis $C_4$-Alkylreste in Betracht. Z.B. stellt $R^7$ einen Methyl-, Ethyl-, Propyl-, Isopropyl-, Butyl-, Isobutyl-, tert.-Butyl-, Pentyl-, 2-Methylbutyl-, Hexyl-, 2-Methyl-pentyl, Heptyl-, Octyl-, 2-Ethylhexyl-, Isooctyl-, Nonyl-, Isononyl-, Decyl-, Isodecyl-, 3,5,5,7-Tetramethylnonyl-, Isotridecyl-, Pentadecyl-, Hexadecyl-oder Octadecylrest dar. Die Bezeichnung Isooctyl, Isononyl, Isodecyl und Isotridecyl sind Trivialbezeichnungen und stammen von den nach der Oxosynthese erhaltenen Alkoholen (vgl. dazu Ullmann, enzyklopädie der Technischen Chemie, 4. Auflage, Band 7, Seiten 216 und 217 sowie Band 11, Seiten 435 und 436).

Bevorzugte 3,4-Dihydro-2H-pyran I sind solche, in denen $R^1$ bis $R^4$ für Wasserstoff oder einen Methylrest stehen und A Methoxycarbonyl, Methoxymethyliden, Methoxymethyl tert. Butoxymethyl, Benzyloxymethyl und Acetoxymethyl bedeutet. Wenn A den Rest $COOR^5$ darstellt, steht $R^3$ vorzugsweise für eine Propyl-, Pentyl-oder Hexylgruppe und insbesondere für Methyl oder Wasserstoff.

Die für das erfindungsgemäße Verfahren benötigten 2-Alkoxy-tetrahydropyrane II können vorteilhaft nach dem in der älteren Anmeldung P 35 36 956.6 vom 17.10.85 beschriebenen Verfahren hergestellt werden.

Die Umsetzung von II zu den 3.4-Dihydropyranen I wird erfindungsgemäß nach einen Verfahren durchgeführt, das in der älteren Anmeldung P 35 07 378.0 (EP 86 102 621.9) vom 02.03.85 allgemein für offenkettige aber auch cyclische Acetale beschrieben ist. Als cyclische Acetal werden lediglich 2,5-Dimethoxy-2,4-dihydrofurane genannt. Die Abspaltung von Methanol führt zum aromatischen Furansystem.

Die Eliminierungsreaktion erfolgte vorteilhaft ohne Verwendung eines Katalysators, d.h. rein thermisch durch Einleiten des 2-Alkoxy-tetrahydropyrans II in flüssigem oder gasförmigem Zustand in eine hochsiedendes Mineralöl, z.B. Gasöl, Vakuumgasöl, schweres Heizöl, technisches Weißöl oder Vakuumrückstand. Die Reaktionstemperaturen liegen oberhalb des Siedepunktes des sich bildenden Dihydropyrans I und betragen im allgemeinen 50 bis 600°C, vorzugsweise 50 bis 550°C, insbesondere 150 bis 350°C.

Im allgemeinen werden Atmosphärendruck oder erhöhter Druck angewendet, es ist aber auch möglich die Umsetzung bei vermindertem Druck durchzuführen. Es können auch die in der älteren Anmeldung angegebenen Katalysatoren in geringen Mengen zugesetzt werden, was aber eher nachteilig ist, das dadurch Nebenreaktionen begünstigt werden können.

Als Reaktoren sind für die Eliminierungsreaktion z.B. Rührkessel geeignet. Vorteilhaft werden jedoch für das Verfahren senkrecht angeordnet zylindrische Reaktoren wie Glockenbodenkolonnen, Blasensäulen oder Füllkörperkolonnen verwendet. Die Tetrahydropyrane II werden in der Regel gasförmig oder flüssig am Boden des mit Mineralöl gefüllten Reaktors zugeführt. Es kann vorteilhaft sein, den verdampften Ausgangsstoff I mit einem inerten Gas zu verdünnen. Geeignete inerte Gase sind z.B. Wasserstoff, Kohlendioxid und vorzugsweise Stickstoff.

Die gebildeten 3,4-Dihydro-2H-pyran I werden gasförmig am Kopf des Reaktors abgezogen. Anschließend werden die gasförmige Produkte zweckmäßig kondensiert. An die Kondensation kann noch eine Reinigungsstufe, z.B. ein Destillation oder Fraktionierung angeschlossen werden.

Das neue Verfahren kann diskontinuierlich oder kontinuierlich nach den dafür üblichen Techniken durchgeführt werden, wobei jedoch die kontinuierliche Arbeitsweise bevorzugt wird. Bei der kontinuierlichen Arbeitsweise kann es vorteilhaft sein, das Mineralöl kontinuierlich zuzuführen und abzuziehen, beispielsweise um gegebenenfalls in geringem Umfang entstandene Nebenprodukte wie Polymere, Crack-oder höhersiedende Nebenprodukte zusammen mit dem Mineralöl aus dem Reaktor auszuschleusen. Eine Aufarbeitung und Rückführung des abgezogenen Mineralöl ist in der Regel nicht wirtschaftlich, da das Mineralöl, z.B. als Heizöl oder Vakuumgasöl in der Regel wohlfeil zur

Verfügung steht. Man wird daher zweckmäßig das mit Nebenprodukten angereicherte Mineralöl einer Verfeuerung und dem Reaktor frisches Mineralöl zuführen.

Das neue Verfahren hat gegenüber demjenigen des Stands der Technik wesentliche Vorteile:

Man benötigt keine sauren Katalysatoren, wodurch Ausgangsstoffe mit säureempfindlichen Resten in hohen Ausbeuten zu den Dihydropyranderivaten I umgesetzt werden können. Gegebenenfalls vorhandene Nebenprodukte verbleiben im Mineralöl, das nicht regeneriert zu werden braucht und das, gegebenenfalls nach Abtrennen des Katalysators, zweckmäßig dem Kraftwerk zugeführt wird.

Die nach dem erfindungsgemäßen Verfahren erhältlichen 3,4-Dihydro-2H-pyranderivative I sind wertvolle Zwischenprodukte für die Synthese von Pharmazeutika oder Farbstoffen und insbesondere von Pflanzenschutzmitteln, z.B. von Herbiziden mit dem Cyclohexan-1,3-dion-grundgerüst, wie sie in der DE-OS 31 21 355 beschrieben sind. Nach Überführung der Gruppe A in die jeweils gewünscht Funktionalität, z.B. in eine Hydroxymethyl-Aldehyd-oder Carboxylgruppe nach literaturbekannten Methoden, können die Zwischenprodukte direkt mit dem Cyclohexandionsystem verbunden werden (vgl. hierzu DE-OS 31 21 355).

Die Erfindung wird in den nachstehenden Beispielen näher erläutert.

Beispiele 1-3

In einer Apparatur gemäß Figur 1 wurde stündlich 60 g an Ausgangsstoff II aus einem Vorratsgemäß 1 über eine Dosierpumpe 2 zusammen mit 200 l/h Stickstoff dem Reaktor 3 über ein Kapillarrohr im flüssiger Form zugeführt. Der Reaktor bestand aus einem Doppelmantelrohr von 1,25 m Länge mit einem Durchmesser von 60 mm. Er war gefüllt mit 1700 g Vakuumgasöl Kp 350°C. Die Reaktionstemperatur betrug 200°C. Die den Reaktor verlassenden Dämpfe werden in den Kühlern 4a, 4b kondensiert und im Austragsbehälter 5 gesammelt. Um gebildete Nebenprodukte wie z.B. Polymere aus dem Reaktor auszutragen wurden stündlich 100 g Vakuumgasöl am Bodenablaßventil ausgeschleust und durch 100 g Frischöl ersetzt.

Die Ergebnisse der Versuche sind in Tabelle 1 zusammengefaßt. Die gebildeten Produkte wurden durch fraktionierende Destillation in einer Reinheit von über 98 % gewonnen.

Tabelle 1

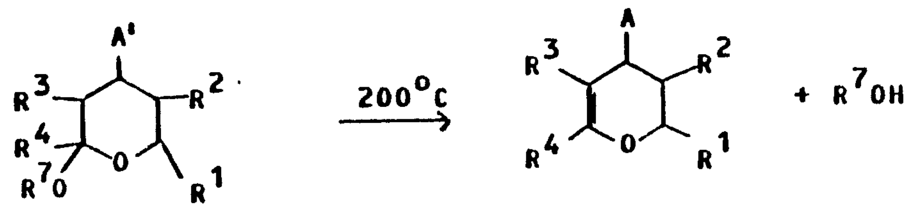


II I

mit $R^1$, $R^3$ und $R^4$ = H; $R^7$=$CH_3$

| Beispiel | Ausgangsstoff II $R^2$ | A' | Menge II/h mol | Austrag g | I im Austrag A | Gew.-% | $CH_3OH$ im Austrag Gew.-% | Ausbeute I %, bez. auf II | Siedepunkt 0°C | mbar |
|---|---|---|---|---|---|---|---|---|---|---|
| 1 | H | $CO_2CH_3$ | 0,34 | 56 | $CO_2CH_3$ | 81,6 | 18,4 | 93 | 68-72 | 10-13 |
| 2 | H | $CH(OCH_3)_2$ | 0,32 | 52 | $=CHOCH_3$ | 66,3 | 33,7 | 87 | 56-62 | 28 |
| 3 | $CH_3$ | $CH_2OOCCH_3$ | 0,32 | 54 | $CH_2OOCCH_3$ | 84,2 | 15,8 | 90 | 96-100 | 10 |

Beispiel 4 (Vergleichsbeispiel zu Beispiel 2)

Ein Gemisch aus 5,0 g (26 mol) 2-Methoxy-4-dimethoxymethyl-terahydropyran und 0,02 g (0,13 mol) p-Toluolsulfonsäure wurde in einen auf 150°C geheizten Kolben getropft. Gleichzeitig wurden 4,4 g des entstandenen Produktgemisches bei 200 mbar abdestilliert, dessen gaschromotographische Menge folgende Zusammensetzung ergab: 60 % 2-Methoxy-4-dimethoxy-methyl-tetrahydropyran, 19 % 4-Methoxymethyliden-3,4-dihydro-2H-pyran und ca. 10 verschiedene strukturell nicht aufgeklärte Nebenprodukte (zusammen 21 %).

## Ansprüche

1. 3,4-Dihydro-2H-pyrane der allgemeinen Formel I

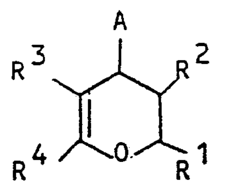

I,

in der die Reste $R^1$ bis $R^4$ gleich oder verschieden sind und für Wasserstoff oder einen $C_1$ bis $C_6$-Alkylrest stehen und A die Reste $COOR^5$ oder $=CH-OR^5$, in denen $R^5$ einen $C_1$-bis $C_4$-Alkylrest darstellt oder A den Rest $CH_2-OR^6$ bedeutet, in dem $R^6$ einen $C_1$-bis $C_4$-Alkylrest, einen Benzylrest, der gegebenenfalls durch einen $C_1$-bis $C_4$-Alkyl- oder Alkoxyrest oder durch Halogen im Phenylring substituiert ist, den Formylrest, einen $C_2$-bis $C_4$-Alkylcarbonylrest oder einen Benzoylrest, der gegebenenfalls durch einen $C_1$-bis $C_4$-Alkyl-oder Alkoxyrest oder Halogen substituiert ist, darstellt mit der Maßgabe, daß $R^3$ nicht für einen $C_2$-oder $C_4$-Alkylrest steht, wenn A den Rest $COOR^5$ bedeutet.

2. Verfahren zur Herstellung von 3,4-Dihydro-2H-pyranen der Formel I gemäß Anspruch 1, durch Abspaltung von Alkoholen $R^7OH$ aus 2-Alkoxy-tetrahydropyranen der Formel II

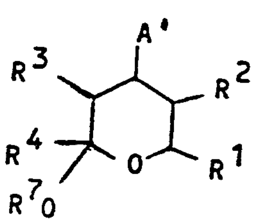

II,

in der die Reste $R^1$ bis $R^4$ gleich oder verschieden sind und für Wasserstoff oder einen $C_1$ bis $C_6$-Alkylrest stehen, A die für Verbindung I genannte Bedeutung hat, A' den Rest $COOR^5$, $CH(OR^5)_2$ oder $CH_2OR^6$ darstellt, wobei die Reste $R^5$ und $R^6$ die für Verbindung I genannte Bedeutung haben und der Rest $R^7$ für einen $C_1$-bis $C_{18}$-Alkylrest steht, dadurch gekennzeichnet, daß man die Abspaltungsreaktion durch Einleiten des 2-Alkoxy-tetrahydropyrans II in flüssigem oder gasförmigem Zustand in ein hochsiedendes Mineralöl bei Temperaturen oberhalb des Siedepunktes des sich bildenden 3,4-Dihydropyrans I durchführt, das Dihydropyran I gasförmig abzieht, das hochsiedende Mineralöl bei Anreicherung mit Nebenprodukten erneutert und das mit Nebenprodukten angereicherte hochsiedende Mineralöl abzieht.

3. Verfahren gemäß Anspruch 2, dadurch gekennzeichnet, daß man die mit Nebenprodukten angereicherten Mineralöle einer Verfeuerung zuführt.

4. Verfahren gemäß Anspruch 2, dadurch gekennzeichnet, daß man als hochsiedendes Mineralöl Gasöl, Vakuumgasöl, schweres Heizöl technisches Weißöl oder Vakuumrückstand verwendet.

5. Verfahren gemäß Anspruch 2, dadurch gekennezeichnet, daß man die Abspaltungsreaktion kontinuierlich durchführt.

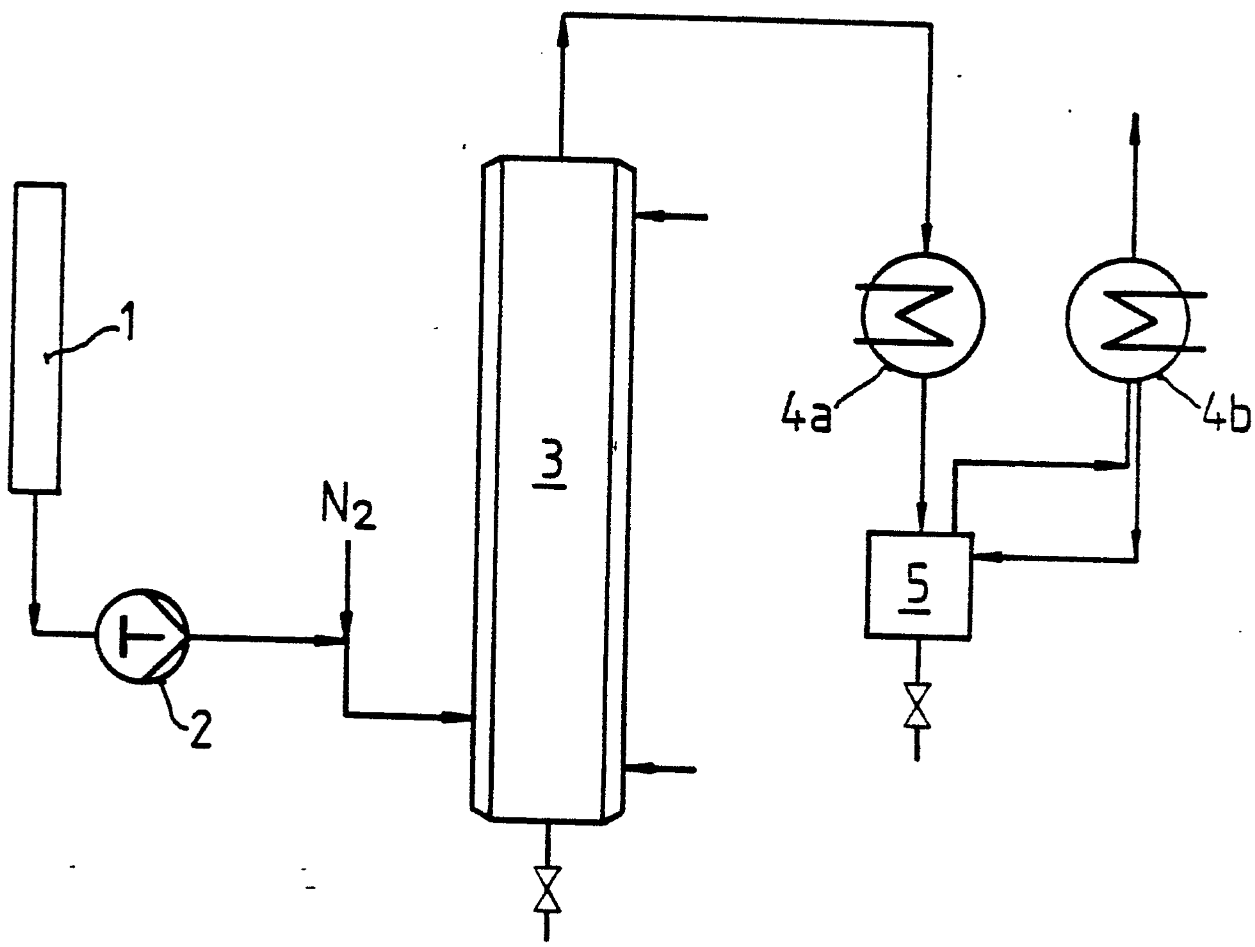
1
2
N2
3
4a
4b
5